# EUROPÄISCHE PATENTSCHRIFT

(11) **EP 1 397 209 B1**
(45) Veröffentlichungstag und Bekanntmachung des Hinweises auf die Patenterteilung: **26.03.2008**
(21) Anmeldenummer: 02730262.9
(22) Anmeldetag: 17.05.2002
(51) Int. Cl.: B01J 23/80, C07C 7/148

(54) **VERFAHREN ZUR REINIGUNG VON ETHYLEN**
PROCESS FOR THE PURIFICATION OF ETHYLENE
PROCÉDÉ POUR LA PURIFICATION D'ETHYLENE

(30) Priorität: 21.05.2001 DE 10124962
(43) Veröffentlichungstag der Anmeldung: 17.03.2004
(73) Patentinhaber: BASF SE, 67056 Ludwigshafen (DE)
(72) Erfinder: VORBERG, Gerald, 67346 Speyer (DE); PÖPEL, Wolfgang, Jürgen, 64297 Darmstadt (DE); MIESEN, Ernest, 67069 Ludwigshafen (DE)
(86) Internationale Anmeldenummer: PCT/EP2002/005470
(87) Internationale Veröffentlichungsnummer: WO 2002/094435

(56) Entgegenhaltungen:
- WO-A-01/70653
- WO-A-02/47818
- DE-A- 4 301 469
- US-A- 3 549 719
- US-A- 4 593 148
- US-A- 4 871 710
- US-A- 5 229 346

## Beschreibung

Die Erfindung betrifft ein Verfahren gemäss Anspruch 1.

Ethylen ist ein wichtiger Rohstoff zur Herstellung von Polyethylen. Bei der großtechnischen Herstellung von Ethylen z.B. durch Naphthaspaltung in einem Steamcracker erhält man ein unreines Ethylen, aus dem die Katalysatorgifte, die die Performance der Polymerisationskatalysatoren - wie aluminiumorganische Ziegler-Natta-Katalysatoren oder chromhaltige Phillipskatalysatoren - beeinträchtigen, entfernt werden müssen (Industrielle organische Chemie, K. Weißermel und H.-J. Arpe, Verlag Chemie, 1976, Seiten 57, 58, 59, 65, 66 und Ziegler-Natta-Catalysts and Polymerisations, J. Boor, Academic Press 1979, Seite 1, 2, 280 bis 285).

Aus Petrochemical Technology Quarterly (PTQ), Seite 103 bis 107 (1997) sind Cu-/ZnO-Katalysatoren zur Reinigung von "feedstock" für Polymerisationen bekannt. Diese Katalysatoren sind bei niedrigen Temperaturen verbesserungsbedürftig und sind gegenüber Acetylenverunreinigungen empfindlich.

Der vorliegenden Erfindung lag daher die Aufgabe zugrunde, den zuvor genannten Nachteilen abzuhelfen.

Demgemäß wurden Katalysatoren für die Reinigung von Ethylen enthaltend Kupfer und Zink, gegebenenfalls einen oder mehrere Promotoren und Träger, hergestellt durch Fällung, Trocknung, Calcinierung und Verpressung gegebenenfalls unter Zusatz von Zuschlagsstoffen gefunden, welche dadurch gekennzeichnet sind, dass man die verpressten Katalysatorteilchen bei 300 bis 700°C nachcalciniert.

Diese Katalysatoren können beispielsweise wie folgt hergestellt werden:

Nach konventionellen Methoden können Nitratlösungen von Kupfer und Zink beispielsweise mit Natriumhydrogencarbonatlösung bei temperaturen von 20 bis 80°C, bevorzugt 25 bis 70°C, besonders bevorzugt 30 bis 60°C, insbesondere 40 bis 55°C gefällt, diese Fällungen mit einer Al₂0₃-Suspension gemischt, gewaschen, angemaischt und sprühgetrocknet, calciniert und in Form (gegebenenfalls mit Zuschlagsstoffen wie beispielsweise Graphit, Talkum, Stearate, Walocel, Stärke, Bortrifluorid) gebracht werden.

Die so erhaltenen geformten Katalysatoren werden einer Nachbehandlung durch Calcinierung bei einer Temperatur von 300 bis 700°C, bevorzugt 350 bis 650°C, besonders bevorzugt 400 bis 600°C, insbesondere 450 bis 580°C in der Regel für 0,5 bis 10 h, bevorzugt 1 bis 3 h, besonders bevorzugt 1 bis 2 h, insbesondere 1 bis 1,5 h unterzogen. Eine besonders bevorzugte Ausführungsform besteht in einem im Verlauf der Verweilzeit ansteigenden Temperaturprofil.

Die so erhaltenen Katalysatoren haben in der Regel eine BET-Oberfläche von 10 bis 100 m²/g, bevorzugt 30 bis 90 m²/g, besonders bevorzugt 40 bis 80 m²/g, insbesondere 50 bis 75 m²/g_{.}

Bei der Herstellung der Katalysatoren erhält man diese in der Regel in "oxidierter" Form, d.h. das Kupfer liegt im Katalysator in Form von Kupferoxid vor. Diese erfindungsgemäßen Katalysatoren können mit Wasserstoff, bevorzugt in einer Wasserstoffatmosphäre bei einer Temperatur von 80 bis 180°C, bevorzugt 100 bis 160°C, besonders bevorzugt 120 bis 140°C und einem Druck von 1 bis 50 bar in ihre "reduzierte" Form überführt werden, wobei das Kupfer darin zumindest teilweise in metallischer Form vorliegt. Eine besondere Ausführungsform besteht darin, dass man die "reduzierte" Form des Katalysators auch in situ, also durch Beimischung ausreichender Mengen Wasserstoff im zu reinigenden Ethylenstrom, erhalten kann.

Die Zusammensetzung der Katalysatoren in "oxidierter" Form kann in weiten Bereichen variiert werden. In der Regel eignen sich solche Katalysatoren, die 30 bis 50 Gew.-%, bevorzugt 35 bis 45 Gew.-%, besonders bevorzugt 40 bis 45 Gew.-% CuO, 30 bis 50 Gew.-%, bevorzugt 35 bis 45 Gew.-%, besonders bevorzugt 40 bis 45 Gew.-% ZnO, 5 bis 40 Gew.-%, bevorzugt 10 bis 30 Gew.-%, besonders bevorzugt 20 bis 30 Gew.-% Al₂O₃, SiO₂, TiO₂, MgO, Eisenoxide oder deren Gemische und 0 bis 5 Gew.-%, bevorzugt 0 bis 2 Gew.-%, besonders bevorzugt 0 bis 1 Gew.-% Promotoren enthalten, bevorzugt daraus, bis auf Spuren, die mit den zuvor genannten Komponenten vergesellschaftet sind, bestehen.

Als Promotoren eignen sich Kalium, Natrium, Mangan, Chrom, Cobalt, Wolfram, Molybdän, Nickel, Eisen, Magnesium, Calcium oder deren Gemische, bevorzugt Kalium, Mangan, Chrom, Molybdän oder deren Gemische, besonders bevorzugt Kalium, Chrom, Molybdän oder deren Gemische.

Die Form und Gestalt der Katalysatoren ist beliebig wählbar wie Tabletten, Ringe, Sterne, Wagenräder, Extrudate wie Zylinder, Pellets oder Stränge, bevorzugt sind Ringtabletten oder Tabletten.

Die Reinigung von Ethylen mit den Katalysatoren kann wie folgt durchgeführt werden:

Das zu reinigende Ethylen kann in einem zweistufigen Prozess
a) in Gegenwart von Wasserstoff an den erfindungsgemäßen Katalysatoren im reduzierten Zustand bei einer Temperatur von 70 bis 110°C, bevorzugt 75 bis 100°C, besonders bevorzugt 80 bis 95°C und einem Druck von 5 bis 80 bar, bevorzugt 10 bis 70 bar, besonders bevorzugt 20 bis 60 bar und anschließend
b) an den erfindungsgemäßen Katalysatoren im oxidierten Zustand bei einer Temperatur von 70 bis 110°C, bevorzugt 75 bis 100°C, besonders bevorzugt 80 bis 95°C und einem Druck von 5 bis 80 bar, bevorzugt 10 bis 70 bar, besonders bevorzugt 20 bis 60 bar umgesetzt werden.

Die Katalysatoren haben eine höhere Resistenz gegenüber Acetylenen, als solche Katalysatoren, die keine erfinderische Nachbehandlung durch Nachcalcinierung der Formkörper erfahren haben. Die erfindungsgemäßen Katalysatoren können mit einen Gehalt von bis zu 200 [ppm] Acetylenen in zu reinigenden Ethylen betrieben werden.

Diesem zweistufigen Prozess kann eine Hydier-Stufe vorgeschaltet sein, die das zu reinigende Ethylen in Gegenwart einer ausreichenden Menge Wasserstoff über einen Hydrierkatalysator, z.B. einen Edelmetallhydrierkatalysator, beispielsweise 0,3 Gew.-% Pd auf einem Al₂O₃-Träger, leitet. Dies ist in der Regel dann angezeigt, wenn im zu reinigenden Ethylen große Mengen Acetylene, also Mengen von mehr als 200 [ppm] enthalten sind.

### Beispiele

### Beispiel 1

### Herstellung des Vergleichskatalysators

Es wird eine Metallnitratmischlösung mit 54 %ige Salpetersäure, Kupfermetall und Zinkoxid hergestellt, wobei das Verhältnis Cu/Zn 100/103 betragen muss. Mit dieser Lösung wird eine Fällung mit 20%-iger Sodalösung in einer Aluminiumhydroxid-Maische durchgeführt. Die so hergestellte Fällmaische wird anschließend auf einer Filterpresse filtriert und gewaschen. Nach Filtration und Waschen des Filterkuchens wurde dieser angemaischt, wieder filtriert und gewaschen [elektrische Leitfähigkeit: unter 150 Mikrosiemens; Nitratgehalt: unter 25 ppm]. Der Filterkuchen wurde mit Wasser zu einer 20 Gew.-%igen Suspension angemaischt, zu einem Pulver sprühgetrocknet und in einem Drehrohr 1 h bei 525°C calciniert. [Nach der Messmethode GV 900 (Glühverlust bei 900°C) betrug der Glühverlust 12 Gew.-%.] Das so erhaltene Pulver wurde unter Zusatz von 1 Gew.-% Graphit zu zylindrischen Tabletten von 5 mm Durchmesser und 3 mm Dicke gepresst. Diese enthielten 40 Gew.-% CuO, 40 Gew.-% ZnO, 19,9 Gew.-% Al₂0₃ und 0,1 Gew.-% K₂0 als Promotor. Das Schüttgewicht betrug 1,2 kg/l, die Porosität 0,3 ml/g, die BET-Oberfläche 125 m²/g.

### Beispiel 2

### Herstellung des erfindungsgemäßen Katalysators

Der so erhaltene konventionelle Katalysator wurde im Drehrohr bei 490°C in der Einlaufzone und 550°C in der Auslaufzone, also bei im Verlauf der Verweilzeit ansteigender Temperatur während 1,2 Stunden calciniert. Das Schüttgewicht betrug 1,24 kg/l, die Porosität 0,34 ml/g, die BET-Oberfläche 64 m²/g.

### Beispiel 3

### Herstellung von Kohlendioxid aus Kohlenmonoxid

Durch eine Testapparatur aus einem adiabat betriebenen Reaktor mit einer Katalysatorfüllung von 450 ml wurde ein Gasgemisch aus 1125 Nl/h Stickstoff und 0,1 Vol.-% Kohlenmonoxid mit einer Raumgeschwindigkeit von 2500 Norm-Liter Gas pro Liter Katalysator und Stunde bei einer Temperatur von 90 bis 120°C geschickt. Am Ausgang wurde das verbliebene nicht umgesetzte Kohlenmonoxid gemessen. Der Umsatz und die benötigte Temperatur sind ein Maß für die Wirkung.

Die Ergebnisse sind nachfolgend zusammengestellt.

### Katalysator nach Beispiel 1

Bei 91°C Reaktionstemperatur fand praktisch keine Oxidation statt, das gesamte CO schlug durch, d.h. es wurde am Ausgang der Testapparatur wiedergefunden. Bei 120°C war der Umsatz nur in den ersten Minuten größer als 99 %, nach 15 Minuten waren im Endgas 90 ppm CO (Umsatz: 91 %) enthalten, nach 10 Stunden schlugen bereits große Mengen durch, der Gehalt betrug 360 ppm (Umsatz: 36 %).

### Katalysator nach Beispiel 2

Bei 91°C Reaktionstemperatur enthielt das Gas Ausgang der Testapparatur auch nach 10 und 18 Stunden nur 9 ppm CO. Der Umsatz lag also auch nach 10 und 18 Stunden bei 99,1 %.

## Patentansprüche

1. Verfahren zur Reinigung von Ethylen unter Verwendung von Katalysatoren, die in oxidierter Form 30 bis 50 Gew.-% CuO, 30 bis 50 Gew.-% ZnO, 5 bis 40 Gew.-% Al₂O₃, SiO₂, TiO₂, MgO, Eisenoxide oder deren Gemische und 0 bis 5 Gew.-% Promotoren enthalten und durch Fällung, Trocknung, Calcinierung und Verformung gegebenenfalls unter Zusatz von Zuschlagstoffen hergestellt werden, wobei man das zu reinigende Ethylen in einem zweistufigen Prozess
a) in Gegenwart von Wasserstoff an einem derartigen Katalysator, in dem das Kupfer zumindest teilweise in metallischer Form vorliegt, bei einer Temperatur von 70 bis 110°C und einem Druck von 5 bis 80 bar und anschließend
b) an einem derartigen Katalysator, in dem das Kupfer in Form von Kupferoxid vorliegt, bei einer Temperatur von 70 bis 110°C und einem Druck von 5 bis 80 bar umsetzt;
**dadurch gekennzeichnet, dass** man Katalysatoren verwendet, die nach der Verformung bei einer Temperatur von 300 bis 700 °C nachcalciniert wurden.

2. Verfahren nach Anspruch 1, **dadurch gekennzeichnet, dass** man Katalysatoren verwendet, die in oxidierter Form 35 bis 45 Gew.-% CuO, 35 bis 45 Gew.-% ZnO, 10 bis 30 Gew.-% Al₂O₃, SiO₂, TiO₂, MgO, Eisenoxide oder deren Gemische und 0 bis 2 Gew.-% Promotoren enthalten.

3. Verfahren nach Anspruch 1 oder 2, **dadurch gekennzeichnet, dass** man Katalysatoren mit einer BET-Oberfläche von 10 bis 100 m²/g verwendet.

## Claims

1. A process for purifying ethylene by using catalysts which in oxidized form comprise from 30 to 50% by weight of CuO, from 30 to 50% by weight of ZnO, from 5 to 40% by weight of Al₂O₃, SiO₂, TiO₂, MgO, iron oxides or mixtures thereof and from 0 to 5% by weight of promoters and which are produced by precipitation, drying, calcination and where pressing, with or without addition of additives, the ethylene to be purified being reacted, in a two-stage operation,
a) in the presence of hydrogen over such a catalyst in which the copper is at least partly present in metallic form, at a temperature of from 70 to 110°C and a pressure of from 5 to 80 bar, and subsequently
b) over such a catalyst in which the copper is present in the form of copper oxide, at a temperature of from 70 to 110°C and a pressure of from 5 to 80 bar;
wherein catalysts used were after pressing subjected to further calcination at a temperature of from 300 to 700°C.

2. The process according to claim 1 wherein catalysts used comprise in oxidized form from 35 to 45% by weight of CuO, from 35 to 45% by weight of ZnO, from 10 to 30% by weight of Al₂O₃, SiO₂, TiO₂, MgO, iron oxides or mixtures thereof and from 0 to 2% by weight of promoters.

3. The process according to claim 1 or 2 wherein catalysts having a BET surface area of from 10 to 100 m²/g are used.

## Revendications

1. Procédé de purification d'éthylène par utilisation de catalyseurs qui contiennent sous forme oxydée 30 à 50 % en poids de CuO, 30 à 50 % en poids de ZnO, 5 à 40 % en poids d'Al₂O₃, de SiO₂, de TiO₂, de MgO, d'oxydes de fer ou leurs mélanges et 0 à 5% en poids de promoteurs et qui sont fabriqués par précipitation, séchage, calcination et mise en forme éventuellement sous addition d'additifs, l'éthylène à purifier étant mis à réagir dans un procédé à deux étapes :
a) en présence d'hydrogène sur un catalyseur de ce type, dans lequel le cuivre est présent au moins partiellement sous forme métallique, à une température de 70 à 110°C et à une pression de 5 à 80 bars et ensuite
b) sur un catalyseur de ce type dans lequel le cuivre est présent sous forme d'oxyde de cuivre, à une température de 70 à 110°C et à une pression de 5 à 80 bars;
**caractérisé en ce qu'**on utilise des catalyseurs qui ont été soumis à une post-calcination après à mise en forme à une température de 300 à 700°C.

2. Procécé selon la revendication 1, **caractérisé en ce qu'**on utilise des catalyseurs qui contiennent sous forme oxydée 35 à 45 % en poids de CuO, 35 à 45 % en poids de ZnO, 10 à 30 % en poids d'Al₂O₃, de SiO₂, de TiO₂, de MgO, d'oxydes de fer ou leurs mélanges et 0 à 2 % en poids de promoteurs.

3. Procédé selon la revendication 1 ou 2, **caractérisé en ce qu'**on utilise des catalyseurs ayant une surface BET de 10 à 100 m²/g.
